# EUROPEAN PATENT SPECIFICATION

(11) **EP 0 831 909 B1**
(45) Date of publication and mention of the grant of the patent: **26.01.2005**
(21) Application number: 96915923.5
(22) Date of filing: 03.06.1996
(51) Int. Cl.: A61K 31/195, A61K 41/00, A61K 49/00, A61P 33/06, A61P 31/10

(54) **USE OF A PROTOPORPHYRIN IX PRECURSOR IN THE TREATMENT OF DERMATOPHYTOSES AND MALARIA AFTER PHOTOACTIVATION OF SAID PROTOPORPHYRIN**
VERWENDUNG EINES PROTOPORPHYRIN IX PRECURSOR ZUR BEHANDLUNG VON DERMATOPHYTOSEN UND MALARIA NACH PHOTOAKTIVIERUNG VON DIESEM BESAGTEN PROTOPORPHYRIN
UTILISATION D'UN PRECURSEUR DE LA PROTOPORPHYRINE IX DANS LE TRAITEMENT DES DERMATOPHYTOSES ET DE LA MALARIA APRES PHOTOACTIVATION DE LADITE PROTOPORPHYRINE

(30) Priority: 05.06.1995 US 465242
(43) Date of publication of application: 01.04.1998
(60) Divisional application: 04028866.4
(73) Proprietor: Queen's University at Kingston, Kingston, Ontario K7L 3N6 (CA)
(72) Inventor: KENNEDY, James, C., Kingston, Ontario K7M 4A6 (CA); POTTIER, Roy, H., Kingston, Ontario K7L 4V1 (CA); REID, Robert, L., Kingston, Ontario K7L 4V1 (CA); SAC-MORALES, Arnold, Kingston, Ontario K7K 6G8 (CA); TOMALTY, Lewis, L., Ontario, Canada K7M 3G7 (CA)
(74) Representative: Simpson, Alison Elizabeth Fraser
(86) International application number: PCT/CA1996/000363
(87) International publication number: WO 1996/039188

(56) References cited:
- WO-A-91/01727
- WO-A-93/20810
- WO-A-94/06424
- WO-A-95/05813
- WO-A-95/07077
- WO-A-95/31189
- DATABASE EMBASE ELSEVIER SCIENCE PUBLISHERS, AMSTERDAM, NL AN=96020997, XP002009953 & NOUVELLES DERMATOLOGIQUES, vol. 14, no. 10, 1995, FRANCE, pages 603-606, SAMSOEN M. : "ARSENAL MEDICAMENTEUX DERMATOLOGIQUE 1995"

## Description

### Background of Invention

Tissue abnormalities involving the skin usually are detected and assessed by a combination of visual inspection and palpation. In certain clinical situations the sensitivity of the visual inspection can be enhanced by the use of non-white light (either ultraviolet or a narrow band in the visible), or by the prior application of a contrast-enhancing agent such as dilute acetic acid or certain stains. Tissues abnormalities that involve surfaces that cannot be palpated (such as the bronchi or the urinary bladder) may be visualized via an appropriate scope. Some specialized scopes can detect induced fluorescence. If the abnormality in question is associated with a difference in either the extent or the pattern of tissue vascularization, such a scope may be used to determine the limits of the area involved by the abnormality, by visualizing an injected bolus of fluorescein or other fluorescent material as it passes through the vasculature of both the lesion and the adjacent normal tissue.

In addition, fluorescence-detecting scopes are being used experimentally to identify areas of tissue that show strong porphyrin fluorescence following the intravenous injection of exogenous porphyrins such as hematophorphyrin IX (HpIX), hematoporphyrin derivative (HpD), or "dihematoporphyrin ether". Such porphyrins tend to accumulate semi-preferentially in malignant tissues, but they also accumulate in tissues that are regenerating following an injury or in the rapidly growing tissues of an embryo or fetus. Normal liver, spleen, and kidney also tend to accumulate these porphyrins. Using such compounds and fluorescence-detecting scopes, areas of malignant tissue too small to be identified by standard forms of visual inspection have been identified in the bronchi and in the urinary bladder.

Unfortunately, a clinically significant (photosensitizing) amount of porphyrin may persist in the skin for at least two weeks, (occasionally for more than two months) following the intravenous injection of HpIX, HpD, or a semi-puridied preparation of HpD, such as Photofrin II. (Photophrin is a registered trademark of Quadra Logics, Inc. Vancouver, British Columbia, Canada.) This means that patients must avoid exposure to sunlight (either direct, or through window glass) for an inconveniently long period of time post-injection. Understandably, patient compliance often is poor, and accidental phototoxic "sunburn" is a common occurrence in the weeks following a diagnostic or therapeutic injection of porphyrin. Persistent photosensitivity is the major hazard associated with this technique, and is the main reason why it is not used more widely.

The standard treatments for cancer comprise surgery, radiotherapy and chemotherapy. However, other forms of treatment are also known, including photochemotherapy or photodynamic therapy (PDT), based on the discovery made over 90 years ago that unicellular organisms, *i*.*e*., certain rapidly growing cells (such as cells of the Lower Kingdom, now referred to as *Protista*), treated with certain chemicals will die when exposed to light. Thus, synthetic porphyrins have been shown *in vitro* to protect cells from infections such as parasites, *e*.*g*., tyromastigotes and sphaeromastigotes of Tyropanosoma cruzi, J. Parasitol., 75(6) 1989, p. 970-976, and gram positive bacteria, mycoplasma and yeasts, Malik *et al*. J. Photochemistry and Photobiology, B. Biology 5 281-293 (1990).

PDT is currently being used, on an experimental basis, to treat several different types of cancer as well as certain non-malignant lesions such as psoriasis. The patient is given a photo-activatable drug that has some degree of specificity for the tissue being treated. A tissue volume that includes the target tissue is then exposed to photoactivating light so as to destroy the target tissue while causing only mild and reversible damage to the other tissues in the same treatment volume.

There are two main types of photochemotherapeutic agents in clinical use at present. The first type, methoxypsoralens, are given systemically. Ultraviolet light is essential to activate them. Localized exposure of psoralen-containing tissues to ultraviolet light induces a localized photochemical reaction that causes the drug to bind covalently to the DNA of living cells, thus destroying their proliferative potential. The second type, porphyrins and related photosensitizers, are also given systemically (by intravenous injection), although occasionally they are given either topically or by intralesional injection. They can be activated by visible (red) light. The localized exposure of porphyrin-containing tissues to such light ordinarily does not induce a chemical reaction between cell components and the porphyrin molecules. Instead, the porphyrins act as catalysts by trapping the energy of the photoactivating light and then passing it on to molecules of oxygen, which in turn are raised to an excited state that is capable of oxidizing adjacent molecules or structures. Cell death is not caused primarily by damage to the DNA, but by damage to essential membrane structures. The goal of photochemotherapy is sometimes cure (mainly for basal cell carcinomas), but usually the goal is palliation through local control when none of the standard forms of therapy are considered likely to offer a significant degree of benefit to the patient.

Methoxypsoralen (PUVA) therapy is used mainly for the treatment of psoriasis, but sometimes it is also used to treat very superficial cancers that involve the skin (mainly mycosis fungoides). However, there are two serious problems with such treatments. First, the procedure has been demonstrated in humans to be carcinogenic. Second, the depth at which malignant tissue can be killed is limited to a few millimeters below the illuminated surface. These problems severely limit the usefulness of the methoxypsoralens for photochemotherapy.

5-Amino-4-oxopentanoic acid, also known as 5-aminolevulinic acid and as δ-aminolevulinic acid ("ALA") has been described in the cross referenced patents and patent applications first set forth in this specification for detecting and treating rapidly growing cells. ALA has also been reported for use in attenuating the growth and killing plants and insects when applied directly to such organisms followed by exposure to light, based on work of Rebeiz *et al*.

Synthetic porphyrins have also been used as photochemotherapeutic agents in treating rapidly growing, e.g. rapidly dividing or rapidly metabolizing infectious cells, such as infectious pathogens, including protozoal parasites, such as *Plasmodium falciparium* (which causes malaria in humans), various other species of *Plasmodia*, *Leishmania,* and amoebae, pathogenic fungi, and mycoplasma including the various parasitic forms, all such cells and organisms being referred to herein as *Protista*. The term *Protista* as used here and in the literature refers to the lowest orders of the animal and vegetable kingdoms, single celled or collections of single celled organisms including: the eukaryotes, including protozoa, fungi and algae, and the prokaryotes, which are bacteria and blue-green algae.

At present, the porphyrins most commonly used for photochemotherapy are Hematoporphyrin IX (HpIX), Hematoporphyrin derivative (HpD) and various semi-purified preparations of HpD such as commercially available Photofrin® II, a semi-purified form of HpD. When porphyrins are used as photosehsitizers, cell death results from damage to cell membranes. Consequently, malignant transformation is not a serious problem. Moreover, since the visible (red) light that is used to photoactivate porphyrins penetrates tissue much more deeply than does the ultraviolet light that must be used to photoactivate methoxypsoralens, the depth at which porphyrin-treated tissue can be killed is substantially greater. Also, since certain types of porphyrins show a significant tendency to accumulate preferentially in malignant tissues, it is sometimes possible to destroy malignant tissue without causing clinically significant damage to adjacent normal tissues.

The main problem with the systemic use of HpIX, HpD and Photofrin II is that photosensitizing concentrations persist in the skin for several weeks to several months following their administration. Consequently, severe accidental phototoxic skin reactions may occur unless the patient avoids exposure to sunlight (either direct, or filtered through window glass) until the concentration of the photosensitizer in the skin has been reduced to a harmless level. At present, the problem of photosensitivity following the administration of porphyrins is handled by advising the patient to avoid any form of exposure to sunlight (or to very bright artificial lights) for a period of at least two weeks post-injection, and to initiate subsequent exposure to sunlight very cautiously. Not all patients comply with these instructions, since it often is quite inconvenient to do so. In addition, the use of a sunscreen with a high blocking factor is recommended with warning that this will only reduce the hazard somewhat, not eliminate it completely. In a few cases, patients whose photosensitization persisted for more than a month post-treatment have been given large daily doses of beta-carotene over a period of several months in an attempt to prevent accidental phototoxic damage. Finally, attempts have been made to reduce phototoxicity by applying the photosensitizer topically to a limited area.

However, another type of problem is encountered if HpIX or HpD is applied topically in DMSO (dimethylsulfoxide), Azone, or some other vehicle intended to enhance their diffusion through tissue. The porphyrins tend to become immobilized wherever they happened to be when the DMSO or Azone becomes diluted by normal tissue fluids to such an extent that the porphyrins can no longer diffuse through the tissue (or even remain in solution). Consequently, the topical application of porphyrins often is associated with a loss of specificity for malignant tissues, and normal tissues near the site of application may develop persistent photosensitization from the localized concentration of porphyrin.

The terms porphyrin(s) and their precursors refer to compounds produced *in vivo* in the synthesis of heme and other endogenously produced photoactivatable compounds including their photoproducts.

The following documents provide useful background to the invention:
WO 95/07077 describes a pharmaceutical composition for the treatment of disorders or abnormalities of external or internal surfaces of the body which are responsive to photochemotherapy, comprising ALA or a precursor thereof, together with at least one surface penetration assisting agent, and optionally one or more chelating agents whereby therapeutic efficacy of the ALA is enhanced.
WO 93/20810 describes medicaments, for detecting and treating malignant and non-malignant tissue abnormalities and lesions of the skin; conjunctiva; respiratory, digestive and vaginal mucosa; endometrium and urothelium; and for ablating the endometrial tissue and treating body fluids containing suspended abnormal cells, and for treating cancers of the nervous system, which are prepared from ALA or precursor thereof and subsequently administered to the patient in an amount sufficient to induce synthesis fluorescence and/or photosensitizing concentrations or protoporphyrin IX in the abnormal cells, followed by exposure of the abnormal cells to light of photoactivating wavelengths.
WO 94/06424 describes a method of destroying or impairing target cells that have selectively accumulated a photosensitizing agent. The target cells are in the bloodstream of an intact animal, which bloodstream and animal further contain non-target cells. Radiation is applied transcutaneously to at least a portion of the intact animal at an intensity effective to impair or destroy selectively the target cells and to leave non-target cells relatively unimpaired. Target cells include leukemia cells, virus-containing cells, parasite-containing cells, and microorganisms such as bacteria, parasites and free viruses.
WO 95/31189 describes a wipe, comprising an absorbent woven or non-woven fabric, cloth or tissue substrate, impregnated with a pharmaceutically active agent, wherein the agent is a substance effective in stimulating melanocytes to produce melanin and/or is effective in a topical treatment of a skin condition in combination with electromagnetic radiation falling in the range of 220-700nm.

### Summary of Invention

The invention is based on the finding that exogenously administered ALA and other precursors of PpIX are metabolized in patients to PpIX and that PpIX preferentially accumulates in rapidly growing cells, as contrasted with less rapidly growing cells. The rapid growth is correlated with the metabolic activity, so that the differential accumulation is affected by the relative metabolic activity between different cells.

There is therefore provided the use of a precursor of protoporphyrin IX for the preparation of a medicament for the treatment of dermatophytoses or malaria in a patient, wherein for the treatment of dermatophytoses following administration of the medicament the patient is exposed to light capable of photoactivating protoporphyrin IX and wherein for the treatment of malaria following administration of the medicament the malarial parasites are exposed to light capable of photoactivating protoporphyrin IX.

In preferred aspects of this invention the preferred precursor of protoporphyrin IX is 5-amino-4-oxo-pentanoic acid, otherwise known as 5-aminolevulinic acid, and a preferred wavelength of the photoactivating light is in the range of 625 to 670 nm, more preferably a red light of 625 to 640 nm.

The dermatophytoses according to the invention include tinea pedis and onyChomycosis, whereas the malarial para-sites include Plasmodium falciparum, Plasmodium ovale, Plasmodium malariae and Plasmodium vivax.

### Detailed Description of the Drawing

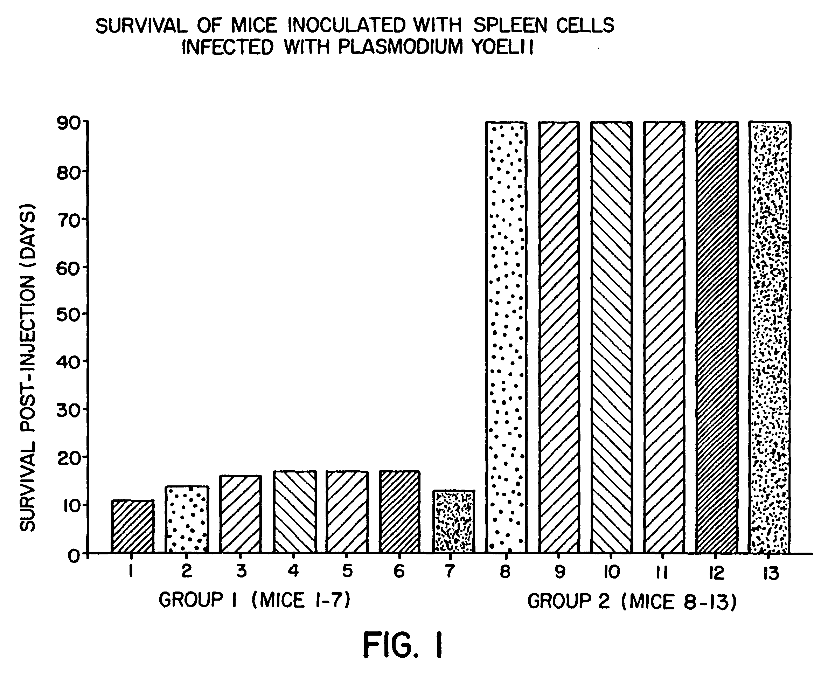

Figure 1 illustrates the duration of survival of individual mice following the injection of spleen cells infected with *P*. *yoelii*. Group (1) mice were given spleen cells that had been exposed to ALA *in vivo* but then kept in the dark. The average survival of the recipients of these cells was 15 days. Group (2) mice were given the same number of cells from the same cell suspension afterit had been exposed to photoactivating light. All of these mice remained in good health for 90 days, at which time the experiment was terminated.

### Detailed Description of Preferred Embodiment

Protoporphyrin IX (PpIX), a naturally occurring photosensitizer, is the immediate precursor of heme in the heme biosynthetic pathway. All nucleated cells have at least a minimal capacity to synthesize PpIX, since heme is necessary for the synthesis of various essential heme-containing enzymes. Certain types of cells and tissues can synthesize relatively large quantities of PpIX. Under normal conditions, the synthesis of PpIX in such tissues is under such tight feed-back control that the cells produce it at a rate just sufficient to match their need for heme. However, the usual rate-limiting step in the process, the synthesis of 5-aminolevulinic acid, can be bypassed by the provision of exogenous ALA, porphobilinogen, or other precursor of PpIX. Certain tissues and organs will then accumulate such a large excess of PpIX that they become both fluorescent and photosensitive. At least in the case of the skin, the PpIX appears to be synthesized *in situ*. ALA, which is commercially available from Sigma Chemical Company and other sources and which is water soluble, can be administered orally, topically or by injection. The oral and parenteral routes lead to the induction of clinically useful concentrations of PpIX in certain benign and malignant tissues throughout the body. 'Only certain types of tissue synthesize and accumulate clinically useful amounts of PpIX when provided with an excess of ALA. By the expression "rapidly growing cell" is meant herein any lesion, abnormal cell or normal cell that exhibits cell growth substantially greater than that of the surrounding tissues and that preferentially accumulates protoporphyrin IX from exogenous ALA. Thus, the cells include rapidly growing cells that are endogenous to the patient and rapidly growing exogenous cells such as Protista and parasite cells. The term "rapidly growing cells" is also used here to include living, metabolically active cells as contrasted with metabolically inactive (dead or dormant) cells such as found in the malarial applications of this invention.

Medicaments prepared by the method of the present invention may be useful for the administration of ALA, to the patient. The administration can also be *in vitro* as applied to tissues of the patient, *i*.*e*., *ex vivo*. In *ex vivo* methods, tissue containing the rapidly growing cells are removed from the patient, an effective amount of ALA or endogenous porphyrin is added thereto, then the preparation is subjected to photoactivating light, before being readministered to the patient. The amounts of ALA constituting an effective dose can be determined by'one skilled in the art by analogy with the doses used for synthetic porphyrins, based on milligrams per kilogram body weight for *in vivo* systemic application and the typical concentrations for topical or *ex vivo* applications. The compound can be conveniently used orally or intravenously at a dosage of about 10 to 100 mg/kg per single dose, preferedly as a dosage of 40-50 mg/kg; however split dosages of 10 mg/kg four times per day may also be given. The compound can be used topically at a dose of between 2% to 100%, with 100% being dry powder. *Ex vivo* concentrations of the compound are used on cell suspensions in a range of 1-5mM, with a preferred range of 1-2mM; however, if serum is present, a higher dose of about 15 mM should be used. If *ex vivo* use on whole blood, the compound is used at about 15 mM; however, if an iron kelator, such as Desferol™ or des ferroxamine, a lower concentration may be used.

Another application of this invention is the killing of parasites preferentially photosensitized by exposure to ALA or an endogenous porphyrin either *in vivo* or *ex vivo*. The conjunctiva, which can be treated either topically or systemically with ALA, followed by, after an appropriate period of time, exposure of the skin or conjuctiva to photoactivating light. The parasites can also be present in the peripheral blood, in which case the ALA can be administered systemically, followed' by, after an appropriate time, which can be easily experimentally determined, exposing the defined area of the skin or the blood passing through a large vein to photoactivating light via an optical guide within a transparent catheter that has been inserted into the vein. Parasites located within one cm. of the surface of hollow organs that are accessible to fiberscopic examination (respiratory tract, digestive tract, urogenital tract, abdominal cavity, pelvic cavity, thoracic cavity) can be diagnosed or treated by systemic administration of the ALA, followed by, after the appropriate period of time, exposure of the surface of the target tissue via an appropriate light guide. Parasites located at sites that are not readily accessible to fiberscopic examination can be treated with the photoactivating light via a light guide that has been surgically introduced into the target area through a needle or following surgery.

Yet another application of this invention is the selective photosensitization and killing of malarial parasites *in vivo* or *in vitro* by exposing them to photoactivating light. The light would be transmitted to the malaria parasites in the circulating blood either through the skin, via an indwelling intravenous or intraarterial catheter or by extracorporeal photodynamic therapy of blood, especially for patients who have failed to respond to other therapies, particularly those who might be considered candidates for a therapeutic exchange transfusion.

The wavelength of the photoactivating light is of some importance, as it has been shown that between 1 and 10 percent of incident red light (600-700 nm) can pass through a slab of human tissue 1 cm thick, whereas only 0.001 percent or less of blue light (about 400 nm) can pass through the same thickness of human tissue. The photosensitizer will, therefore, be more successful if it absorbs red light. PpIX does strongly absorb red light. The present approach has several advantages over the prior art. First endogenous PpIX has a much shorter half-life in normal tissues (human and mouse, at least) than does HpIX, HpD or Photofrin® II. This greatly reduces the danger of accidental phototoxic skin reactions in the days following treatment. Second, the ALA can be applied topically to certain types of lesions. This improves the specificity of the treatment, reduces the danger of accidental phototoxic reactions to a very low level, and greatly reduces the amount of both ALA and PpIX to which the entire body would be exposed if an equally effective dose of ALA were to be given systemically.

Both ALA and PpIX are normal products of metabolism, and are handled quite readily by the biochemical machinery of the body. However, since very large doses of ALA (like large doses of HpIX or HpD) are associated with a transient decrease in motor nerve conduction velocity, it is desirable to reduce the dose of ALA to the minimum that is still effective. Topical application requires much less ALA than systemic administration. Third, PpIX is rapidly inactivated by the photoactivating light. Following exposure of tissues containing PpIX to a therapeutic dose of photoactivating light, there is a substantial decrease in photosensitization of the tissues within the treatment volume. Consequently, if PpIX is induced by the topical application of ALA to specific lesions, the patient can be exposed to sunlight immediately post-treatment without danger of serious phototoxicity. Also, the dosimetry of the photoactivating light is great simplified. Fourth, ALA is an effective inducer of PpIX when given by mouth, by topical application, or by injection. In contrast, HpIX, HpD and Photofrin II are effective in most situations only when given by injection. The versatility of ALA enhances its acceptability for routine use by the medical profession, since the oral and topical routes of administration are much more convenient than the parenteral. Fifth, the normal and abnormal tissues that can be photosensitized by the administration of ALA are somewhat different from those that can be photosensitized by the administration of HpIX, HpD or Photofrin II. Consequently, ALA would be useful in clinical situations in which the other photosensitizers are not.

### EXAMPLE 1

### (1) Selective induction of the synthesis and accumulation of protoporphyrin IX and/or other endogenous porphyrins within parasites in vivo or in vitro.

*In vivo* - If the parasites in question involve the skin, conjunctiva, oral mucosa, nasal mucosa, anal mucosa, or urothalium, ALA may be applied directly to the surface of the affected tissue. If the parasites' are located at sites that are not suitable for topical application, an effective amount of ALA is administered systemically, either by mouth, by subcutaneous injection, or by intravenous injection.

*In vitro -* The material suspected of containing parasites is incubated under appropriate conditions in the presence of an effective concentration (generally around 5 mM) of ALA.

### EXAMPLE 2

### IN VIVO STUDIES

The injection of an effective dose of 5-aminolevulinic acid (ALA) into mice infected with *P*. *yoelii* leads to the accumulation of fluorescing and photosensitizing concentrations of protoporphyrin within metabolically active parasites. There is no such accumulation of protoporphyrin within non-viable parasites, or within normal erythrocytes or leukocytes. In parasitized erythrocytes, the protoporphyrin accumulation is localized to the parasite itself.

Metabolically active (viable) malarial parasites can be distinguished readily from parasites that are inactive (dead), since only parasites that are metabolically active can synthesize protoporphyrin. In addition, metabolically active (viable) malarial parasites can be killed selectively by exposing infected blood or cell suspensions to photoactivating wavelengths of light. This procedure causes no significant damage to the accompanying normal erythrocytes and leukocytes, since they do not accumulate enough protoporphyrin to become photosensitized.

### EXAMPLE 3

### Demonstration, Quantification, and Analysis of ALA-Induced Fluorescence Within Erythrocytes Parasitized by P. yoelii

Normal mice were given intraperitoneal injections of blood or spleen cells obtained from mice infected with *P*. *yoelii*. When the malaria was well established, some of the infected mice were given a single intraperitoneal injection of 250 mg of ALA per kg of body weight. Controls included infected mice that were not given ALA, and non-infected mice that were given/not given ALA.

At various intervals thereafter, suspensions of blood and/or spleen cells were examined by the following techniques.

Fluorescence Microscopy: Red fluorescence developed within parasitized erythrocytes of mice given ALA, but not within any of the controls. This fluorescence was localized to the plasmodia.

Fluorescence Flow Cytometry: Large numbers of erythrocytes in suspensions of cells from the peripheral blood and spleen of heavily parasitized mice given ALA developed red fluorescence. Cells from the control mice were uniformly negative. This technique permitted the rapid detection and enumeration of erythrocytes that contained metabolically-active parasites, and produced relative values for the intensity of ALA-induced fluorescence in such erythrocytes.

Spectrophotofluorometry: Blood and spleen cells from heavily parasitized mice given ALA were washed and pelleted by centrifugation. Protoporphyrin was the only fluorophore that was identified by spectrophotofluorometry. As expected, cell pellets from the control animals contained only traces of protoporphyrin.

### Demonstration and Quantitation of ALA-Induced Photosensitization of the Intra-Erythrocytic Stage of P. yoelii.

Normal mice were given intraperitoneal injections of blood or spleen cells obtained from mice infected with *P*. *yoelii*. When the malaria was well established, some of the infected mice were given a single intraperitoneal injection of 250 mg of ALA per kg of body weight. Controls included infected mice that were not given ALA, and non-infected mice that were given/not given ALA.

At various intervals thereafter, suspensions of blood and/or spleen cells were exposed to graded doses of photoactivating light. Light-induced loss of viability of the *P. yoelii* was demonstrated by (a) loss of infectivity, or (b) loss of ability to accumulate the fluorescent cleavage product of calcein-AM.
(A) Infectivity Assay: Mice infected with *P. yoelii* were given a standard dose of ALA by intraperitoneal injection. Peripheral blood and/or spleen cells were collected after a standard interval, exposed to standard doses of photoactivating light (including a no-light control) and then injected into normal mice. If the control (no-light) mice developed malaria and died while the mice given cells that had been exposed to a given dose of light remained free of malaria and lived indefinitely, this was considered to be evidence that the light treated cell suspensions did not contain enough viable plasmodia to cause an infection.
   For example, a Balb/c mouse with advanced malaria (*P*. *yoelli*) was given an intraperitoneal injection of 250 mg of ALA per kg of body weight. Four hours later, its spleen cells were suspended in isotonic saline. Half of the spleen cell suspension was placed on ice and ekxposed to photoactivating light (waveband 600-700 nm, intensity 100 nW/cm², total dose 540 J/cm²), while the other half was kept on ice in the dark. Balb/c mice were injected intraperitoneally with either the light treated or untreated sample. Survival of the mice was followed for 90 days. Figure 1 illustrates the duration of survival of individual mice following the injection of spleen cells infected with *P*. *yoelii*.
(B) Photosensitization studies (*Ex vivo* studies, direct photoradiation): A group of 4 hairless female mice were used. Two mice were infected with *P*. *yoelii* and 2 other mice were non-infected. Mice infected with malaria were usually in the 8th day following inoculation with plasmodia. Mice were divided in two groups: one group was treated with ALA, the control group was not treated with ALA.
   Both groups were then kept in the dark for a period of 3 hours. Mice were then sacrificed (overdoses of chloroform) and infected blood cells were obtained from homogenized spleen. Spleens were homogenized in 3cc of isotonic saline solution. From this homogenization 1cc was taken and diluted in 24cc of isotonic saline solution, then from this dilution 1cc was taken and placed in test tubes (a total of 8 tubes). Four tubes were kept in dark and four tubes were photoirradiated.
   The source of light was a tungsten lamp with a filter for red light (600-700 nm). The beam was 10 cm in diameter and the fluence about 70 mW/cm2. The samples were placed in ice on a turntable (33 rpm) to assure a uniform distribution of the light in the target cells.
   To determine the viability of the plasmodium after being irradiated, the contents of each tube were inoculated into hairless mice, and then the mice were followed for survival.
   Control groups for light alone but not ALA, and ALA but not light, were also used to make sure that photsensitization was due to ALA plus light.
(C) Spectrophotofluorimetric studies: A group of 8 hairless 1 female mice were used. Four mice were in the 8th day post inoculation with *Plasmodium yoelii* with 35% parasitemia and 4 normal mice were normal (non-infected). The mice were divided into 4 groups of two mice in each.
   i) 2 infected mice were given an IP injection of 250 mg/kg of ALA in PBS.
   ii) 2 normal (non-infected) mice were also injected IP with 250 mg/kg of ALA in PBS.
   iii) 2 reference controls were included: 2 infected mice with malaria and 2 non-infected mice, none received ALA.

   All 4 groups were kept at normal room temperature, in the dark, for 4 hours and then sacrificed. Mice were anesthetized with chloroform and then blood was collected by cardiac puncture (heparinized syringe with 20G 1' needle). Approximately 0.9 cc blood was collected and transferred to a 5 cc test tube, kept on ice and in the dark. Test tubes were then centrifuged for 10 minutes. Using a spectrophotofluorometer set for excitation at 410 nm and fluorescence emission at 635 nm., fluorescence measurements were taken of the supernatant and the pellet. Hemolysis with 1% saponin was carried out in samples after the first fluorescence measurements, and the free Plasmodia are centrifuged to form a pellet. Then fluorescence measurements were taken from the pellet and the supernatant. Protoporphyrin fluorescence was detected only in Plasmodial pellet derived from infected mice given ALA.
(D) Flow cytometer studies (Pharmacokinetic studies): A group of 4 hairless female mice were used. Two mice were infected with *P. yoelii* and 2 other mice were not infected. Mice infected with malaria were usually in the 8th day post inoculation with the infected plasmodia. ALA was given directly to the mice (250 mg/kg intraperitoneal), then 2 drops of whole blood were withdrawn at regular intervals of time from the tail of each mouse and placed in 5 cc flow cytometer test tubes containing 0.5 cc of RPMI 1640 and then analyzed by the flow cytometer to follow accumulation of PpIX. Only infected mice given ALA developed fluorescence in their erythrocytes.

For the *in vitro* studies, no ALA was given to the donor mice. Two drops of whole blood were withdrawn from the tail of each mouse and placed in a 35 mm petri dish containing 3 cc of RPMI 1640 without phenol red.
i) a petri dish contained infected whole blood with 5nM ALA in RPMI.
ii) a second petri dish contained infected whole blood plus RPMI but not ALA.
iii) a third petri dish contained normal whole blood cells with 5mM ALA in RPMI.
iv) a fourth petri dish contained normal whole blood cells with RPMI but not ALA.

All petri dishes were incubated at 37 Celsius and room air environment. Samples (0.5 cc) were taken at regular intervals from these incubated petri dishes to be analyzed in the flow cytometer to follow accumulation of PpIX. Only cells from infected mice developed PpIX fluorescence when incubated with ALA.

### Application of ALA-Induced PpIX PDT to the Treatment of Malaria.

Malaria is caused by infection of the host with unicellular parasites known as plasmodia. At one stage in their life cycle, the plasmodia infect and develop within erythrocytes of the peripheral blood, spleen, and/or marrow. They may infect the liver and certain other organs also.

Of the numerous species of plasmodia that have been identified, only a few can infect humans. Plasmodia that cause malaria in mice but not humans provide a safe and convenient model for laboratory studies of malaria.

These examples involve the murine malarial parasites *Plasmodium yoelii* (lethal strain) and *Plasmodium chabaudi* (non-lethal strain) as models for human malaria.

### IN VIVO PHOTOSENSITIZATION

When mice infected with the murine malarial parasites *P. yoelii* or *P*. *chabaudi* were given an adequate dose of 5-Aminolevulinic Acid (ALA) by intraperitoneal injection,
- what appeared spectroscopically to be protoporphyrin (PpIX) accumulated in many of the plasmodia within erythrocytes of the peripheral blood, spleen, and marrow. However, significant concentrations of PpIX did not accumulate within the non-infected erythrocytes or within the great majority of the leukocytes in the infected mice.
- a fluorescent material that may have been a complex of protoporphyrin with a light metal (perhaps zinc protoporphyrin) sometimes accumulated in association with the PpIX.
- following exposure to an adequate dose of light of wavelengths within the photoactivation spectrum of PpIX, the plasmodia that had been exposed to ALA lost their normal ability to accumulate calcein when exposed to calcein-AM, and also lost their ability to cause malaria when injected into recipient mice. However, the non-infected erythrocytes and the leukocytes in the same cell suspensions showed no morphological evidence of damage following exposure to the photoactivating light.

### IN VITRO PHOTOSENSITIZATION

When peripheral blood, spleen, or bone marrow cells from mice infected with the murine malarial parasites *P*. *yoelii* or *P*. *chabaudi* were incubated under suitable conditions in the presence of an effective concentration of ALA, what appeared spectroscopically to be protoporphyrin (PpIX) accumulated within many of the plasmodia in erythrocytes of the peripheral blood, spleen, or marrow. However, significant concentrations of PpIX did not accumulate within the non-infected erythrocytes or within the great majority of the leukocytes in the infected mice.

The exposure of metabolically active *P*. *yoelii* or *P*. *chabaudi* to an effective concentration of ALA under suitable conditions *in vivo* or *in vitro* leads to the preferential accumulation of fluorescing and photosensitizing concentrations of PpIX in those plasmodia, but not in non-infected erythrocytes or in the great majority of the leukocytes in peripheral blood, spleen, or bone marrow cell suspensions.

Plasmodia-specific ALA-induced fluorescence can be used to detect and quantitate metabolically active malarial parasites in suspensions of cells from peripheral blood, spleen, or marrow.

Plasmodia-specific ALA-induced photosensitization can be used to destroy malarial parasites selectively, by exposing them *in vitro* or *in vivo* to an adequate dose of photoactivating light.

### EXAMPLE 4 Cutaneous Fungal Infections

Historically, fungal infections have not attracted as much attention as bacterial infections. This focus of research has been due to a number of factors,' most notably, the high incidence, the degree, and the effect of bacterial infections in humans. However, this trend has changed in the past couple of decades. With the increasing number of immunocompromised patients, both by iatrogenic (chemotherapy) and disease (AIDS) causes, the incidence of fungal infections has increased. This has coincided with an increase in the morbidity and mortality rates due to fungal infections in the last decade.

Fungal infections can be divided into three categories: cutaneous, subcutaneous and systemic. While the systemic infections (*blastomycosis, candidiasis,* etc.) have more serious sequelae, the cutaneous infections are much more prevalent. Between 1971 and 1974, fungal infections had a reported rate of 88/1000 persons in the U.S. with the non-invasive cutaneous infections responsible for 90% of the cases. (This is the number of reported cases. Because of the non-life threatening sequelae of cutaneous infections, the actual incident rate is likely much higher.) They were also cited as the most common skin infection.

Cutaneous infections can be further divided into three sub-categories: superficial, dermatophytoses and dermatomycoses. Superficial infections do not penetrate the outer layer of the skin and do not involve either the hair or nails. *Tinea nigra, black piedra* and *white pedra* are examples of superficial fungal infections. Dermatophytoses are infections of the skin, hair, and nails, and include all layers of the stratum corneum. These infections are caused by dermatophytes, fungi which rarely cause disseminated infections. These organisms release keratinases, which likely explains their localization within the keratinized tissues. These fungi cause little mortality, but are a major cause of morbidity worldwide, and in North America a major expenditure of time and money. These infections predispose their hosts to bacterial superinfections. Dermatomycoses are cutaneous infections caused by-non-dermatophytes and have a greater chance of invasion and dissemination (e.g.. superficial candidiasis, mycetoma, sporotrichosis), especially in an immunocompromised host. However, as stated before, the greater majority of fungal infections are caused by the non-invasive dermatophytes.

### Dermatophytes

Dermatophytes include *Trichophyton* spp., *Microsporum* spp. and *Epidermophyton* spp. genera. Ecologically, these fungi are anthrophilic (human to human transmission), zoophilic (animal to human transmission) and geophilic (soil to human transmission, possibly via an animal intermediary). Typically the anthrophilic fungi cause little inflammation (increasing the likelihood of chronic infection) and the zoophilic fungi cause a furuncular reaction.

Dermatophytoses are named "tinea" followed by the body location (e.g., *tinea capitis* is an infection of the head). Table 1 lists the dermatophytoses and their causative dermatophyte as found in a survey of dermatological visits by U.S. Army personnel. This data has been supported by data collected from surveys of students, inmates, and other armed forces personnel in the U.S. The most common dermatophyte worldwide is *T*. *rubrum* (survey of major dermatologic centers).

**Table 1**

| Incidence of Dermatophytoses and the Causative Dermatophytes | | |
|---|---|---|
| Dermatophytoses | Incidence | Most common Dermatophytes (in L to R) |
| tinea pedis | 44% | *T*. *mentagrophytes*. *T*. *rubrum* |
| tinea unguium | 16% | *T*. *rubrum, T*. *mentagrophytes, E*. *floccosum* |
| tinea cruris | 15% | *T*. rubrum, *T*. *mentagrophytes, E*. *floccosum* |
| tinea corpis | 13% | *T*. *Rubrum* |
| tinea barbae | 4% | *T. mentagrophytes, T. verrucosum* |
| tinea capitis | 3% | *T*. *tonsurans, M. canis* |

### Clinical Presentation

These infections are not life threatening but they can cause a significant amount of discomfort. Typically they cause scaling, fissuring, peeling, itching, burning erythema, and in some circumstances, maceration. Tinea capitis usually causes reversible hair loss. *T*. *mentagrophytes* and *T. verrucosum* can produce a violent inflammatory reaction. As well, these infections are not pretty and can have serious aesthetic consequences. The outcome of these infections is either a spontaneous cure, a cure by medication, a treatable chronic condition, or a persistent infection despite medication. Both the presentation and outcome is a function of the dermatophyte virulence and the host's defense capabilities. Immunocompromised individuals invariably fare worse than their immunocompetent counterparts.

### Treatment

Dermatophytoses can be treated topically or orally. The advantage of treating topically is that more aggressive (toxic) therapy can be employed, whereas orally, less toxic drugs are required. However, topical drugs can cause itching, burning, redness, and sensitization of the infected area. Oral therapy has the advantage of gaining access to tissue sites normally unattainable to topical therapy (i.e. the nail beds). To gain access to the site of action, both routes must overcome the body's natural defenses to foreign molecules since none of the drugs used are endogenous molecules. The imidazoles and triazoles are used topically and ketoconazole and griseofulvin orally. However, ketoconazole has a large number of side effects, especially if used for a long period of time, and *T*. *rubrum* and *T. tonsurans* have shown resistance to therapy. Both oral regimens require careful monitoring and some patients may not be treated because of contraindications.

Antifungal therapy depends on the thickness of the site infected. *Tinea cruris* and *corpis* require a shorter treatment time than *tinea manum* and *tinea pedis* because the skin is thinner in the groin and on the body as compared to the hands and feet. Infections localized to the hair follicle roots require 4 to 6 weeks of treatment (root = 3-4 mm under the skin surface, at 1 mm/ week growth). The fingernails require 4-9 months of treatment, and the toenails, which grow even slower, require 9-18 months of treatment. Due to wearing shoes, the feet and toenails are also subjected to an environment which is conductive to fungal growth (warm, moist), making it more difficult to eliminate the infection.

*Tinea unguium* or *onychomycosis* has been particularly troublesome to treat. Treatment regimens can last as long as 18 months, with considerable time and money invested in the cure. Nail avulsion (removal) is often included in the regimen but may cause considerable postoperative discomfort. Even so, only a 75-80% cure rate can be obtained with fingernail infections. The results are more bleak for toenail infections (25% cure rate). If more than one nail is involved, a permanent cure is unlikely. It has been estimated that at least 15-20% of the U.S. population between the ages of 40-60 have *onychomycosis.*

### Clinical application of ALA-induced photosensitization to chronic toenail infection with dermatophyte (Trichophyton species)

An adult male presented with a chronic dermatophytic infection involving the nail of the great toe. The nail itself was badly deformed as a result of the infection. The surrounding tissues showed evidence of chronic low-grade inflammation.

A 20% (w/w) solution of 5-aminolevulinic acid (ALA) in an oil-in-water emulsion (Glaxal Base) was applied to the toenail and surrounding tissues, and then covered with a water-resistant plastic dressing (Tegaderm). Four hours later, the Tegaderm and residual cream were removed and the whole area exposed to photactivating (red) light.

The patient experienced a typical subjective response while the toe was being exposed to the light - itching, stinging, and a sensation of mild burning. Upon completion of treatment, the toe was erythematous and somewhat edematous. This gradually decreased over the next few days.

Over the next few months, all clinical evidence of the fungal infection vanished. The toenail is now growing without deformity.

### EXAMPLE 5

The following organisms accumulate fluorescing and/or photosensitizing concentrations of PpIX when exposed to exogenous ALA:
(1) Protozoa
   (a) *Leishmania - L. donovani*
      [ALA-induced fluorescence]
   (b) Malaria - *Plasmodium yoelii*
      [ALA-induced fluorescence]
      [ALA-induced photosensitization] *- Plasmodium chaubadi*
      [ALA-induced fluorescence]
      [ALA-induced photosensitization]
(2) Worms
   (a) Nematodes - *Lumbricus terrestris* (dewworm)
      [ALA-induced fluorescence]
      [ALA-induced photosensitization] *- Enterobius vermicularis* (pinworm)
      [ALA-induced fluorescence]
      [ALA-induced photosensitization]

*Plasmodium yoelii* is a malarial parasite that can infect and grow progressively to produce a lethal form of malaria in susceptible strains of mice and rats. The inventors have found that, when normal mice are injected with standard numbers of blood or spleen cells obtained from donors infected with *P. yoelii*, they die of malaria 10 to 20 days after such injection. This mouse model is applicable to the study of malarial infections in humans, including *P*. *vivax, P. falciparum, P*. *malariae*, and *P*. *ovale*.

## Claims

1. The use of a precursor of protoporphyrin IX for the preparation of a medicament for the treatment of dermatophytoses or malaria in a patient, wherein for the treatment of dermatophytoses following administration of the medicament the patient is exposed to light capable of photoactivating protoporphyrin IX and wherein for the treatment of malaria following administration of the medicament the malarial parasites are exposed to light capable of photoactivating protoporphyrin IX.

2. The use as claimed in Claim 1 wherein said precursor of protoporphyrin IX is 5-aminolevulinic acid.

3. The use as claimed in Claim 1 or Claim 2 wherein said dermatophytoses are selected from tinea pedis or onychomycosis.

4. The use as claimed in Claim 1 or Claim 2 wherein said malarial parasites are selected from Plasmodium falciparum, Plasmodium ovale, Plasmodium malariae or Plasmodium vivax.

## Patentansprüche

1. Verwendung eines Protoporphyrin IX-Präkursors zur Herstellung eines Medikaments zur Behandlung von Dermatophytose oder Malaria bei einem Patienten, wobei bei der Behandlung von Dermatophytose der Patient nach der Verabreichung des Medikaments Licht ausgesetzt wird, das das Protoporphyrin IX photo aktivieren kann, und wobei bei der Behandlung von Malaria die Malariaparasiten nach der Verabreichung des Medikaments Licht ausgesetzt werden, das das Protoporphyrin IX photoaktivieren kann.

2. Verwendung nach Anspruch 1, wobei der Protoporphyrin IX-Präkursor 5-Aminolävulinsäure ist.

3. Verwendung nach Anspruch 1 oder Anspruch 2, wobei die Dermatophytose aus Fußpilz oder Nagelpilzwucherung ausgewählt ist.

4. Verwendung nach Anspruch 1 oder Anspruch 2, wobei die Malariaparasiten aus Plasmodium falciparum, Plasmodium ovale, Plasmodium malariae oder Plasmodium vivax ausgewählt sind.

## Revendications

1. Utilisation d'un précurseur de protoporphyrine IX pour la préparation d'un médicament destiné au traitement de dermatophytoses ou du paludisme chez un patient, dans laquelle, pour le traitement de dermatophytoses, après administration du médicament, le patient est exposé à de la lumière capable de photo-activer la protoporphyrine IX, et dans laquelle, pour le traitement du paludisme, après administration du médicament, les parasites responsables du paludisme sont exposés à de la lumière capable de photo-activer la protoporphyrine IX.

2. Utilisation suivant la revendication 1, dans laquelle ledit précurseur de protoporphyrine IX est l'acide 5-aminolévulinique.

3. Utilisation suivant la revendication 1 ou la revendication 2, dans laquelle lesdites dermatophytoses sont choisies entre la teigne des pieds et l'onychomycose.

4. Utilisation suivant la revendication 1 ou la revendication 2, dans laquelle lesdits parasites responsables du paludisme sont choisis entre *Plasmodium falciparum, Plasmodium ovale, Plasmodium malariae* et *Plasmodium vivax*.
